# EUROPEAN PATENT SPECIFICATION

(11) **EP 0 481 290 B1**
(45) Date of publication and mention of the grant of the patent: **11.12.1996**
(21) Application number: 91116837.5
(22) Date of filing: 02.10.1991
(51) Int. Cl.: A61B 5/0402, A61B 5/0408, H01B 7/08

(54) **Cable for connecting an electrocardiograph to limb electrodes and chest electrodes**
Anschlusskabel für Gliedmassen- und Brustelektroden an einen Elektrokardiographen
Câble pour connecter les électrodes des membres et du buste à un électrocardiographe

(30) Priority: 10.10.1990 IT 1516190 U
(43) Date of publication of application: 22.04.1992
(73) Proprietor: MORTARA RANGONI EUROPE S.r.l., 40016 San Giorgio di Piano (Bologna) (IT)
(72) Inventor: Zini, Roberto, I-48022 Lugo di Ravenna (IT)
(74) Representative: Porsia, Bruno

(56) References cited:
- DE-A- 3 346 866
- US-A- 4 353 372

## Description

The present invention relates to a cable for connecting an electrocardiograph instrument to limbs electrodes and chest electrodes placed on the body of a patient, comprising a multiple core strap formed by a number of single core leads which are flexible and each being provided with a screen and arranged in a coplanar way adjacent to each other in substantial mutual contact and interconnected by at least one longitudinal continuous web of the same plastic material as that forming an external insulating sheath of the leads, this strap being provided at one end with a terminal with a connector in a housing for removable connection to the corresponding socket of the electrocardiograph, means of protection of the instrument, such as voltage dischargers, being incorporated in the housing of the connector, while at the other end of the strap the individual leads are branched off from the strap and - having been previously cut to the required length - are provided at their free ends with means for rapid and removable connection with the electrodes, each lead being associated with an electric resistance for protection against excess voltages.

A cable of this type is known from the document US-A- 4 353372. In this known cable, the individual leads are branched off one after the other from the strap or all together from the end of the strap, without reinforcing the branching points, and the means provided at the free ends of the branched off individual leads for connection with the electrodes are formed as pads.

Document DE-A-3 346 866 discloses an ECG-multilead cable where clamps are used in branching off points for the leads but with quite different electrical connections proximally and distally and unscreened leads

The invention aims to provide a connecting cable of the aforesaid type which is more economical, easier to use and more reliable than the known type of cable.

The cable according to the invention is characterized by the combination of the following features:
a) at an intermediate point the strap is incorporated in a first clamp, after which the individual leads for the limbs electrodes are branched off,
b) at an end point of the remaining strap, this strap is incorporated in a second clamp, after which the individual leads for the chest electrodes are branched off,
c) the means provided at the free ends of the individual leads for connection with the electrodes, are constituted of plugs, each provided with an incorporated electrical resistor.

The characteristics of the improved cable in question, and the advantages derived from it, will be evident from the following description which refers to the figures on the single attached sheet of drawings, in which:
Figure 1 is schematic plan view of the new connecting cable;
Figure 2 shows a magnified partial view in transverse section along the line II-II in figure 1 of the multiple-core and multiple-screen strap forming the new connecting cable; and
Figure 3 shows the terminal for connecting the new cable to the electrocardiograph, in longitudinal section along the line III-III in figure 1.

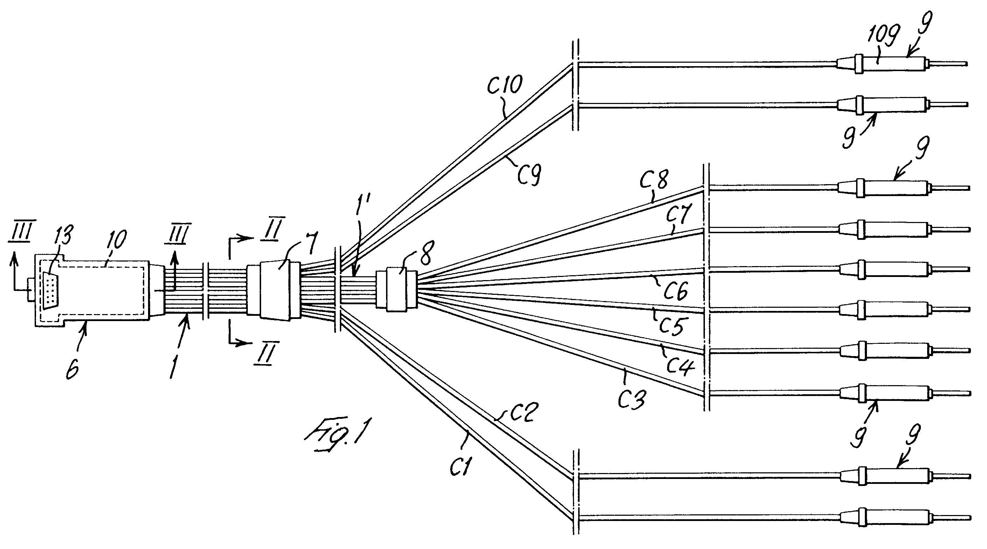

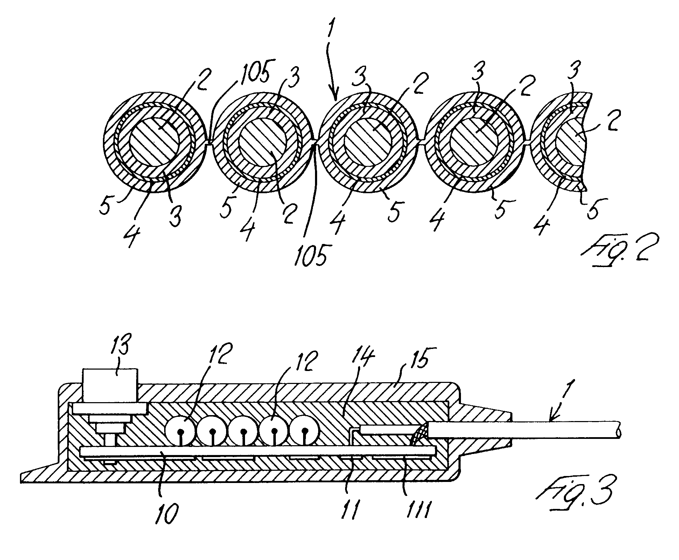

With reference to figures 1 and 2 initially, it will be noted that the new connecting cable is provided with a number of electrical conductors which are adjacent, flexible, parallel, and normally equidistant from each other 2, each of which is provided with at least one first flexible insulating sheath 3, then a flexible screening braid 4 and at least one flexible outer covering 5. The conductors, the screening and the insulating sheaths are made from any suitable materials having high technological reliability, such that they impart to the leads a considerable degree of flexibility and considerable resistance to mechanical stresses and to chemical and physical attack in general. The external sheaths 5 of the various leads are spaced a short distance apart, substantially in mutual contact, and are interconnected in a coplanar way by continuous webs 105 of suitable thickness, of the same material as that of the said sheaths, such as to form a multiple-core strap 1, containing the desired number of individually screened leads. The strap 1, formed in this way, may be constructed by known methods of extrusion of the plastic material forming the sheaths 5 and the connections 105.

The new cable uses a section of multiple-core strap 1 of predetermined length, comprising for example 10 individually screened leads, which is provided at one end with a connector 6 described subsequently, for removable connection to the electrocardiograph. At the point at which the branch connections to the electrodes for the upper and lower limbs of the patient are to branch off, said strap is enclosed in a small flat body 7, having the function of a clamp, formed for example by injection of a suitable plastic material, preferably of the same type as that forming the sheaths 5. The leads for connection to the limbs are adjacent to each other and may be located on the same side of the strap or, as in the present example, on both sides of said strap. The leads for connection to the limbs branching from the clamp 7, indicated by C1-C2 and C9-C10, are mutually separated by breaking the web 105 connecting them together and to the other six leads from C3 to C8 which form the section of strap 1' which leaves the clamp 7 and remain interconnected.

At the point at which the individual connections for the precordial branches are to be taken off from the last section 1' of the strap, in other words for connection to the electrodes placed on the patient's chest, the strap is enclosed in a small flat body 8, having the function of a clamp, also formed by injection of suitable plastic material. Leads C3 to C8 which are branched from the clamp 8 are separated from each other by breaking the interconnecting web 105, whose consistency is such that no substantial trace of the web remains, also to give maximum free sliding of the external sheath 5 of the individual leads.

The individual leads branched from the clamps 7 and 8 are cut to the necessary length and their free ends are connected to the plugs 9 for rapid and removable connection to the measurement electrodes attached to the patient's body. The connection to the plugs 9 shall be such that the necessary metallic continuity of the conductor 2 and of the screen 4 of each lead is ensured, and includes the connection of an electrical resistor of suitable value in series with each conductor, to limit the risk of excess voltages in the direction of the electrocardiograph, for example in combined use with pacemakers, defibrillators or other electrical medical equipment. The body 109 of each plug 9, which covers the electrical connections to the lead and the aforesaid electrical resistor, is preferably formed by injection of a suitable plastic material, the same as in the clamps 7 and 8, to prevent tampering with the plugs and to ensure a sealed connection between the sheath of the lead and the metal terminals of the plug.

The composition of the terminal 6 for connection to the electrocardiograph will now be described, with reference to the detail in figure 3. This figure shows that this component comprises a printed-circuit base 10, provided at one end with points 11 for selective connection to the electrical conductors of the strap 1 and with one or more points 111 close to the points 11 and preferably external, for connection to the screening 4 of the leads constituting the strap, which have previously been bundled together. The voltage dischargers 12 are mounted on the intermediate part of the base 10, and the multiple-core connector 13 to be inserted into the electrocardiograph socket is connected electrically and mechanically to the other end of the said base. The base 10, together with the components 12, and already connected to the strap 1 and to the connector 13, is then placed in a small mould into which is injected epoxy resin 14 which rapidly solidifies and incorporates the parts described, including an end section of the strap 1 and part of the body of the connector 13 which is thus securely fixed to the base. This may make it possible to carry out only the electrical connection of the connector to the base 10 beforehand.

The resin body 14 is then housed in another mould into which is injected the same plastic material as that which forms the clamps 7 and 8, to provide the terminal in question with a coating 15 of more suitable material which ensures a sealed connection between the metal part of the connector 13 and the sheaths 5 of the leads forming the strap 1. Like the plugs 9, the terminal 6 cannot be tampered with.

It is to be understood that the terminal 6, with the connector 13, may have a plan different from the T shape illustrated in figure 1, and that the connector may be formed and oriented differently depending on the type of connection used in the electrocardiograph.

The simplicity of construction, the considerable economy and the considerable technological reliability of the connecting cable described are evident at this point, and are due to the continuity of the screening of the individual leads forming the strap used, permitting the elimination of the complex and expensive junction box used in known types of connecting cable. If appropriate terminals 9 and 13 are used, the new connecting cable is also completely sealed, a condition not present in known connecting cables, partly due to the internal accessibility of said junction box.

## Claims

1. Cable for connecting an electrocardiograph instrument to limbs electrodes and chest electrodes placed on the body of a patient, comprising a multiple core strap (1) formed by a number of single core leads (C1-C10) which are flexible and each being provided with a screen (4) and arranged in a coplanar way adjacent to each other in substantial mutual contact and interconnected by at least one longitudinal continuous web (105) of the same plastic material as that forming an external insulating sheath (5) of the leads, this strap (1) being provided at one end with a terminal (6) with a connector (13) in a housing for removable connection to the corresponding socket of the electrocardiograph, means of protection of the instrument, such as voltage dischargers (12), being incorporated in the housing (15) of the connector (13), while at the other end of the strap (1) the individual leads (C1-C10) are branched off from the strap (1) and - having been previously cut to the required length - are provided at their free ends with means for rapid and removable connection with the electrodes, each lead (C1-C10) being associated with an electric resistance for protection against excess voltages,
characterized by the combination of the following features:
a) at an intermediate point the strap (1) is incorporated in a first clamp (7), after which the individual leads (C1, C2, C9, C10) for the limbs electrodes are branched off,
b) at an end point of the remaining strap (1'), this strap (1') is incorporated in a second clamp (8), after which the individual leads (C3-C8) for the chest electrodes are branched off,
c) the means provided at the free ends of the individual leads (C1-C10) for connection with the electrodes, are constituted of plugs (9), each provided with an incorporated electrical resistor.

2. Cable according to claim 1, characterized in that the individual leads (C1, C2, C9, C10) for the limbs electrodes are branched off for one half on one side and for the other half on the other side of the strap (1).

3. Cable according to claim 1, characterized in that all the individual leads (C1, C2, C9, C10) for the limbs electrodes are branched off on the same side of the strap (1).

4. Cable according to claim 1, characterized in that both clamps (7, 8) are formed by injection of the same type of plastic material as that forming the external insulating sheath (5) of the leads.

5. Cable according to claim 1, characterized in that the bodies (109) of the plugs (9) at the free ends of the individual leads are formed by injection of the same type of plastic material as that forming the external insulating sheath (5) of the leads.

## Patentansprüche

1. Anschlußkabel für die an dem Körper eines Patienten angebrachten Gliedmassen- und Brustelektroden an ein Elektrokardiographie-Gerät, enthaltend ein mehradriges Band (1), das aus einer Anzahl von einzelnen Kernleitungen (C1 - C10) hergestellt ist, die flexibel und jeweils mit einer Abschirmung (4) versehen und in koplanarer Weise benachbart zueinander im wesentlichen in Kontakt stehend angeordnet sind und durch mindestens einen in Längsrichtung verlaufenden kontinuierlichen Steg (105) des gleichen Kunststoffmaterials verbunden ist, wie dasjenige, aus dem ein äußerer Isoliermantel (5) der Leitungen besteht, wobei dieses Band an dem einen Ende mit einer Anschlußeinheit (6) versehen ist, die in einem Gehäuse einen Stecker (13) zum lösbaren Anschließen an die entsprechende Steckdose des Elektrokardiographen enthält, wobei Einrichtungen zum Schutz des Elektrokardiographie-Instrumentes, wie beispielsweise Spannungsableiter (13), in das Gehäuse (15) des Steckers (13) eingearbeitet sind, während an dem anderen Ende des Bandes (1) die einzelnen Leitungen (C1 - C16) von dem Band (1) abgezeigt sind und - nachdem sie vorher auf die erforderliche Länge zugeschnitten worden sind - an ihren freien Enden mit Einrichtungen zum schnellen und lösbaren Anschließen an die Elektroden versehen sind, wobei jeder Leitung (C1 - C10) zum Schutz gegen Überspannungen ein elektrischer Widerstand zugeordnet ist,
gekennzeichnet durch die Kombination der folgenden Merkmale:
a) das Band (1) ist an einem Zwischenabschnitt in eine erste Klemme eingefügt, nach der die einzelnen Leitungen (C1, C2, C9, C10) für die Gliedmassenelektroden abzweigen,
b) an einem Endpunkt des verbleibenden Bandes (1') ist dieses Band (1') in eine zweite Klemme (8) eingefügt, hinter der die einzelnen Leitungen (C3 - C8) für die Brustelektroden abzweigen,
c) die Einrichtungen an den freien Enden der einzelnen Leitungen (C1 - C10) zum Anschließen an die Elektroden sind durch Stöpsel (9) gebildet, von denen jeder mit einem zugeordneten elektrischen Widerstand versehen ist.

2. Kabel nach Anspruch 1, dadurch gekennzeichnet, daß die einzelnen Leitungen (C1, C2, C9, C10) für die Gliedmassenelektroden zu einer Hälfte an einer Seite und zur anderen Hälfte an der anderen Seite des Bandes (1) abgezweigt sind.

3. Kabel nach Anspruch 1, dadurch gekennzeichnet, daß sämtliche einzelnen Leitungen (C1, C2, C9, C10) für die Gliedmassenelektroden an der gleichen Seite des Bandes (1) abgezweigt sind.

4. Kabel nach Anspruch 1, dadurch gekennzeichnet, daß beide Klemmen (7, 8) durch Spritzgießen des gleichen Typs von Kunststoffmaterial wie das Material hergestellt sind, welches den äußeren Isoliermantel (5) der Leitungen bildet.

5. Kabel nach Anspruch 1, dadurch gekennzeichnet, daß die Körper (109) der Stöpsel (9) an den freien Enden der einzelnen Leitungen durch Spritzgießen des gleichen Typs von Kunststoffmaterial wie das Material gebildet sind, welches den äußeren Isoliermantel (5) der Leitungen bildet.

## Revendications

1. Câble destiné à connecter un instrument d'électrocardiographie à des électrodes de membres et des électrodes de buste placées sur le corps d'un patient comprenant une nappe de conducteurs multiples (1) formée d'un certain nombre de fils à conducteur simple (C1 à C10) qui sont souples et qui sont chacun munis d'un blindage (4) et agencés d'une façon coplanaire et contigus l'un à l'autre en contact mutuel substantiel et interconnectés par au moins une bande continue longitudinale (105) de la même matière plastique que celle qui forme une gaine isolante externe (5) des fils, cette nappe (1) étant munie à une extrémité d'une prise (6) comportant un connecteur (13) dans un boîtier, destiné à une connexion amovible à l'embase correspondante de l'électrocardiographe, un moyen de protection de l'instrument, tel que des dispositifs de décharge de tension (12) qui sont incorporés dans le boîtier (15) du connecteur (13), alors qu'à l'autre extrémité de la nappe (1) les fils individuels (C1 à C10) se séparent à partir de la nappe (1) et, ayant été précédemment coupés à la longueur requise, sont munis à leurs extrémités libres de moyens destinés à une connexion rapide et amovible avec les électrodes, chaque fil (C1 à C10) étant associé à une résistance électrique destinée à la protection contre des tensions excessives,
caractérisé par la combinaison des caractéristiques suivantes :
a) au niveau d'un point intermédiaire, la nappe (1) est incorporée dans un premier collier de serrage (7), après lequel se séparent les fils individuels (C1, C2, C9, C10) destinés aux électrodes des membres,
b) au niveau d'un point d'extrémité de la nappe restante (1'), cette nappe (1') est incorporée dans un second collier de serrage (8), après lequel se séparent les fils individuels (C3 à C8) destinés aux électrodes de buste,
c) les moyens prévus aux extrémités libres des fils individuels (C1 à C10) destinés à la connexion avec les électrodes, sont constitués de fiches (9), chacune étant munie d'une résistance électrique incorporée.

2. Câble selon la revendication 1, caractérisé en ce que les fils individuels (C1, C2, C9, C10) destinés aux électrodes des membres, se séparent pour une moitié d'un côté et pour l'autre moitié de l'autre côté de la nappe (1).

3. Câble selon la revendication 1, caractérisé en ce que tous les fils individuels (C1, C2, C9, C10) destinés aux électrodes des membres se séparent du même côté de la nappe (1).

4. Câble selon la revendication 1, caractérisé en ce que les deux colliers de serrage (7, 8) sont formés par injection du même type de matière plastique que celle formant la gaine isolante externe (5) des fils.

5. Câble selon la revendication 1, caractérisé en ce que les corps (109) des fiches (9) aux extrémités libres des fils individuels sont formés par injection du même type de matière plastique que celle formant la gaine isolante externe (5) des fils.
